# EUROPEAN PATENT APPLICATION

(11) **EP 3 750 977 A1**
(43) Date of publication of application: **16.12.2020**
(21) Application number: 19751754.3
(22) Date of filing: 25.01.2019
(51) Int. Cl.: C11B 9/00, A23L 27/20, A61K 8/31, A61K 8/34, A61K 8/35, A61K 8/37, A61K 8/49, A61Q 13/00, C11D 3/50

(54) **MODULE AROMA MIXTURE**

(30) Priority: 08.02.2018 JP 2018020746
(71) Applicant: Shiseido Company, Ltd., Chuo-ku Tokyo 104-0061 (JP)
(72) Inventor: NAKAMURA, Yasuko, Yokohama-shi, Kanagawa 224-8558 (JP); KAWAZOE, Hiroko, Yokohama-shi, Kanagawa 224-8558 (JP); HIRAO, Naoyasu, Yokohama-shi, Kanagawa 224-8558 (JP); MANAKA, Yuta, Yokohama-shi, Kanagawa 224-8558 (JP); UEDA, Kaoru, Yokohama-shi, Kanagawa 224-8558 (JP)
(74) Representative: Ter Meer Steinmeister & Partner
(86) International application number: PCT/JP2019/002563
(87) International publication number: WO 2019/155921

(57) **Abstract**

The present invention provides a module aroma mixture capable of offering a feeling of happiness while maintaining the aromatic note of a main aroma component when added to a cosmetic or food/beverage. The module aroma mixture according to the present invention to be used in combination with a main aroma component is a module aroma mixture including hexyl acetate and limonene, and also including at least one type of compound selected from the group consisting of isoamyl acetate, ethyl 2-methylpentanoate, trans-2-hexen-1-ol, alpha-damascone, cis-3-hexen-1-ol, linalool, ethyl acetate, ethyl 2-methylacetate, nonanal, neral, neryl acetate, geranial, geranyl acetate, citronellol, nerol, geraniol, ethyl propionate, hexanal, ethyl hexanoate, rose oxide, hexanol, cis-3-hexenyl acetate, methyl anthranilate, diethyl malonate, ethyl decanoate, octanal, decanal, ethyl 2-methylpentanoate, 2-tert-butylcyclohexyl acetate, and dimethylphenethylcarbinyl acetate, wherein from 0.05 to 5% by mass of the module aroma mixture is blended relative to a total amount of the module aroma mixture and the main aroma component.

## Description

### Cross Reference to Related Applications

The present application claims priority to Japanese Patent Application No. 2018-20746 filed February 8, 2018, the entire contents of which are incorporated herein by reference.

### Technical Field

The present invention relates to a module aroma mixture, and particularly, to a module aroma mixture capable of offering a feeling of happiness while maintaining the aromatic note of a main aroma component when added to a cosmetic or food/beverage.

### Background Art

Products such as foods/beverages and cosmetics usually include various aromas. The image of a product changes depending on the fragrance thereof, and therefore, the selection of the aroma to be added is important.

In recent years, fragrance imparting agents including various aromas have been developed for changing the fragrance of products. For example, Patent Literature 1 discloses an alcoholic-feel imparting agent that imparts an alcoholic feel to a non-alcoholic beverage.

However, there has yet to be developed an aroma capable of imparting a feeling of happiness to a product with little or no fragrance that offers a feeling of happiness.

### Citation List

### Patent Literature

Patent Literature 1: Japanese Unexamined Patent Publication No. 2014-45712A

### Summary of Invention

### Technical Problem

The present invention has been made in view of the aforementioned conventional art, and a problem to be solved is the provision of a module aroma mixture capable of offering a feeling of happiness while maintaining the aromatic note of a main aroma component when added to a cosmetic or food/beverage.

### Solution to Problem

As a result of diligent research to solve the aforementioned problem, the Inventors have found that it is possible to obtain a module aroma mixture including a specific aroma component that, by blending from 0.05 to 5% by mass of the module aroma mixture relative to the total amount of a main aroma component, a feeling of happiness can be offered while maintaining the aromatic note of the main aroma component when added to a cosmetic or food/beverage, thus accomplishing the present invention.

More specifically, a module aroma mixture according to the present invention to be used in combination with a main aroma component is a module aroma mixture including hexyl acetate and limonene, and also including at least one type of compound selected from the group consisting of isoamyl acetate, ethyl 2-methylpentanoate, trans-2-hexen-1-ol, alpha-damascone, cis-3-hexen-1-ol, linalool, ethyl acetate, ethyl 2-methylacetate, nonanal, neral, neryl acetate, geranial, geranyl acetate, citronellol, nerol, geraniol, ethyl propionate, hexanal, ethyl hexanoate, rose oxide, hexanol, cis-3-hexenyl acetate, methyl anthranilate, diethyl malonate, ethyl decanoate, octanal, decanal, ethyl 2-methylpentanoate, 2-tert-butylcyclohexyl acetate, and dimethylphenethylcarbinyl acetate, wherein from 0.05 to 5% by mass of the module aroma mixture is blended relative to a total amount of the module aroma mixture and the main aroma component.

In this module aroma mixture to be used in combination with a main aroma component, the blending amount of hexyl acetate and limonene in the module aroma mixture is from 5 to 40% by mass within the total amount of module aroma composition.

Preferably, this module aroma mixture is capable of offering a feeling of happiness.

### Advantageous Effects of Invention

The present invention can provide a module aroma mixture capable of offering a feeling of happiness while maintaining the aromatic note of a main aroma component when added to a cosmetic or food/beverage.

### Brief Description of Drawings

[Fig. 1] Fig. 1 is a diagram illustrating the degree of each sample's effect of producing a happy feeling (facial expression analysis).
[Fig. 2] Fig. 2 is a diagram illustrating results of evaluating the impression of each sample's fragrance (happiness) (sensory evaluation).

### Description of Embodiments

The module aroma mixture according to the present invention can, by being used in combination with a main aroma component, offer a feeling of happiness while maintaining the aromatic note of the main aroma component when a specific amount is added to a cosmetic or food/beverage.

The module aroma mixture needs to include hexyl acetate and limonene as essential components.

In addition to the aforementioned essential components, it is necessary to include at least one type of compound selected from the group consisting of isoamyl acetate, ethyl 2-methylpentanoate, trans-2-hexen-1-ol, alpha-damascone, cis-3-hexen-1-ol, linalool, ethyl acetate, ethyl 2-methylacetate, nonanal, neral, neryl acetate, geranial, geranyl acetate, citronellol, nerol, geraniol, ethyl propionate, hexanal, ethyl hexanoate, rose oxide, hexanol, cis-3-hexenyl acetate, methyl anthranilate, diethyl malonate, ethyl decanoate, octanal, decanal, ethyl 2-methylpentanoate, 2-tert-butylcyclohexyl acetate, and dimethylphenethylcarbinyl acetate.

It is preferred that the blending amount of hexyl acetate and limonene is from 5 to 40% by mass within the total amount of module aroma composition. If the blending amount of hexyl acetate and limonene is less than 5% by mass, there may be cases where it is difficult to impart a feeling of happiness. Also, if the blending amount of hexyl acetate and limonene exceeds 40% by mass, there may be cases where it is difficult to impart a feeling of happiness.

The blending amount of the module aroma mixture needs to be from 0.05 to 5% by mass relative to the total amount of the module aroma mixture and the main aroma component. If the blending amount is less than 0.05% by mass, there may be cases where it is impossible to impart a feeling of happiness. If the blending amount of the module aroma mixture exceeds 5% by mass, there may be cases where the aromatic note is affected.

The main aroma component may include, for example, a citrus note, floral note, fruity note, or green note, although not particularly limited thereto. Particularly, it is preferred to include a floral and/or fruity note. Using a main aroma component having a citrus note, floral note, fruity note, or green note may allow the effects of the module aroma mixture of the present application to become more pronounced.

Further, it is preferred to include 99 wt% or greater, more preferably from 99 to 99.5 wt%, of an aroma having a citrus note, floral note, fruity note, and/or green note within the total amount of the main aroma component.

Products to which the module aroma mixture is to be blended are not particularly limited, with examples including cosmetics, foods/beverages, etc. Examples of cosmetics include: hair cosmetics such as shampoos, conditioners, hair treatments, hair oils, hair creams, pomade, and hair fragrances; makeup cosmetics such as lipsticks and face powders; and skincare cosmetics such as toners, emulsion, serums, body creams, and body gels.

Examples of foods/beverages include: soft drinks such as carbonated beverages, fruit juice beverages, vegetable beverages, isotonic drinks, soymilk, vitamin supplementary drinks, mineral supplementary drinks, energy drinks, lactic acid bacteria beverages, and milk beverages; taste-oriented beverages such as green tea, oolong tea, black tea, herbal tea, tea with milk, and coffee beverages; alcoholic beverages such as *shochu*-based beverages, cocktails, low-malt beers/sparkling liquors, and fruit wines; dairy products such as cow's milk, butter, cheese, and yogurt; desserts such as ice cream, yogurt, pudding, and jello; confectionery such as candies, caramels, crackers, biscuits, cookies, pies, chocolates, and snack foods; and general food products such as bread, soup, and precooked foods.

Examples of other products include: detergents such as soap, laundry detergents, antiseptic detergents, and deodorizing detergents; fragrance products such as perfumes, room fragrances, and car air fresheners; and other products such as toothpastes, tissue paper, and toilet paper.

Methods for producing products including the module aroma mixture are not particularly limited; the module aroma mixture of the present invention may be blended at any discretionary stage in a method for producing a product. For example, a product including a main aroma component may be produced first, and then the module aroma mixture may be added thereto.

Products including the module aroma mixture can produce a fragrance offering a greater feeling of happiness compared to products that do not include the module aroma mixture.

### Examples

The present invention is described in further detail according to the following examples, although the present invention is not limited thereto. The blending amount is indicated in percent by mass (mass%) unless specifically indicated otherwise.

First, the evaluation test methods employed in the present invention will be described before describing the working examples.

### Evaluation (1): Facial expression analysis.

Sixty-four general panelists performed the evaluation test. Each panelist verified whether or not she felt a sense of happiness when she smelled each of four types of fragrances. The panelist's facial expression when she smelled each fragrance and talked about that fragrance was video-recorded. The facial expression at that time was used to verify whether or not the degree of happiness (HAPPY), as objectively and quantitatively estimated from the facial expression, differed depending on the respective fragrances. The facial expression was video-recorded; in terms of which facial expression is to be used for the analysis, each panelist checked the recorded video himself/herself after the measurement, and selected, for each fragrance, the facial expression that most clearly showed the impression of the fragrance. From the visual image of the facial expression for each of the four types of fragrances selected by each panelist, the facial expression was analyzed by using FaceReader (from Noldus Information Technology), which is capable of objectively and quantitatively analyzing facial expression from a visual image of the face and evaluating the state of emotion. This method was performed according to the test described in Japanese Patent Application No. 2018-10593.

Evaluation (2): Sensory evaluation (preference, happy image, and image corresponding to the same aromatic note).

Sixty-four female panelists smelled each sample, and, for each sample, evaluated her preference and whether or not it was possible to perceive a happy image and an image corresponding to the same aromatic note, according to the following criteria.
{Preference}
4: Like.
3: Somewhat like.
2: Cannot say either way.
1: Somewhat dislike.
0: Dislike.
{Happy image; Image corresponding to the same aromatic note}
5: Can be perceived.
4: Can be perceived somewhat.
3: Cannot say either way.
2: Cannot be perceived somewhat.
1: Cannot be perceived.

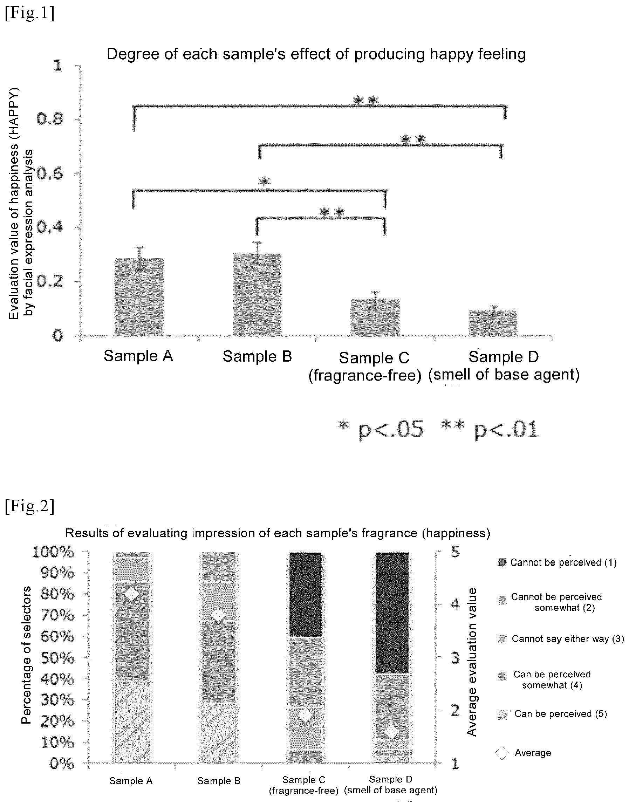

According to the aforementioned evaluation criteria (1) with sixty-four expert panelists, the Inventors evaluated the following fragrances: a shampoo base agent (sample D) not including any of the following aromas; sample A and sample B respectively including 1 mass% of the following aroma composition (A, B) to dipropylene glycol; and fragrance-free sample C (100% dipropylene glycol). The results are shown in Fig. 1. As for the statistical testing method, an analysis of variance was conducted for the values relatively calculated by FaceReader (from Noldus Information Technology) from the selected visual images, and after verifying the differences in values depending on the respective fragrances, multiple comparison was conducted according to Bonferroni's test.

### Base agent:

Sodium methyl cocoyl taurate: 6.6 (mass%);
Cocamidopropyl betaine: 6.3;
Water: 87.1.

### Aroma composition A:

2-Acetyl-2,3,8,8-tetramethyl-1,2,3,4,5,6,7,8-octahydronaphthalene: 13 (mass%);
1,3,4,6,7,8-Hexahydro-4,6,6,7,8,8-hexamethylcyclopenta-y-2-benzopyran: 10;
Methyl dihydrojasmonate: 8;
Limonene: 6;
Linalyl acetate: 5;
Hexyl acetate: 4;
Benzyl benzoate: 4;
Others: 50.

### Aroma composition B:

### {Aroma components}

Limonene: 25 (mass%);
Hexyl acetate: 15;
Ethyl 2-methylacetate: 10;
Ethyl 2-methylpentanoate: 10;
Linalool: 10;
2-Tert-butylcyclohexyl acetate: 5;
Geranyl acetate: 5;
Citronellol: 5;
Dimethylphenethylcarbinyl acetate: 5;
Alpha-damascone: 2;
Geraniol: 5.

### {Solvent}

Dipropylene glycol: 3.

Fig. 1 shows that samples A and B each offered a significant feeling of happiness.

The Inventors prepared samples (samples P, Q, and R) by: respectively blending 0%, 0.5%, or 1% of the aforementioned aroma composition B, as the module aroma mixture, to the aforementioned aroma composition A as the main aroma component (fruity/floral-base aroma composition); and diluting each blend into a 1% dipropylene glycol solution. Each sample was evaluated according to the aforementioned evaluation criteria (2). The results are shown in Table 1.

**[Table 1]**

| | Sample P | Sample Q | Sample R |
|---|---|---|---|
| Fruity/floral-base | 100 | 99.5 | 99 |
| Module aroma mixture | 0 | 0.5 | 1 |
| Preference | 2.92 | 2.88 | 3.04 |
| Happy image | 2.76 | 3 | 2.88 |
| Image corresponding to the same aromatic note | 2.81 | 2.88 | 2.91 |

The samples Q and R, respectively obtained by adding 0.5 to 1% of the module aroma mixture to the sample P, tended to be able to offer a happy image, compared to the main aroma sample P. The compositions obtained by mixing the module aroma mixture to the main aroma sample P resulted in offering an image that is substantially identical to the image of the composition consisting only of the main aroma component, thereby demonstrating that the aromatic note can be maintained.

This thus shows that, by blending the module aroma mixture to the main aroma component, a feeling of happiness can be imparted while maintaining the aromatic note.

The results also revealed that there is no correlation between preference and a feeling of happiness.

The results of the study by the Inventors revealed that it is possible to obtain a feeling of happiness while maintaining the aromatic note of the main aroma component by blending from 0.05 to 5% by mass of the module aroma mixture relative to a total amount of the module aroma mixture and the main aroma component, wherein the module aroma mixture includes hexyl acetate and limonene, and also includes at least one type of compound selected from the group consisting of isoamyl acetate, ethyl 2-methylpentanoate, trans-2-hexen-1-ol, alpha-damascone, cis-3-hexen-1-ol, linalool, ethyl acetate, ethyl 2-methylacetate, nonanal, neral, neryl acetate, geranial, geranyl acetate, citronellol, nerol, geraniol, ethyl propionate, hexanal, ethyl hexanoate, rose oxide, hexanol, cis-3-hexenyl acetate, methyl anthranilate, diethyl malonate, ethyl decanoate, octanal, decanal, ethyl 2-methylpentanoate, 2-tert-butylcyclohexyl acetate, and dimethylphenethylcarbinyl acetate.

## Claims

1. A module aroma mixture, to be used in combination with a main aroma component, wherein:
the module aroma mixture comprises hexyl acetate and limonene; and
the module aroma mixture comprises at least one type of compound selected from the group consisting of isoamyl acetate, ethyl 2-methylpentanoate, trans-2-hexen-1-ol, alpha-damascone, cis-3-hexen-1-ol, linalool, ethyl acetate, ethyl 2-methylacetate, nonanal, neral, neryl acetate, geranial, geranyl acetate, citronellol, nerol, geraniol, ethyl propionate, hexanal, ethyl hexanoate, rose oxide, hexanol, cis-3-hexenyl acetate, methyl anthranilate, diethyl malonate, ethyl decanoate, octanal, decanal, ethyl 2-methylpentanoate, 2-tert-butylcyclohexyl acetate, and dimethylphenethylcarbinyl acetate, wherein from 0.05 to 5% by mass of the module aroma mixture is blended relative to a total amount of the module aroma mixture and the main aroma component.

2. The module aroma mixture, to be used in combination with a main aroma component, according to claim 1, wherein:
the blending amount of hexyl acetate and limonene in the module aroma mixture is from 5 to 40% by mass within the total amount of module aroma composition.

3. The module aroma mixture, to be used in combination with a main aroma component, according to claim 1 or 2, wherein:
the module aroma mixture is capable of offering a feeling of happiness.
